# EUROPÄISCHE PATENTANMELDUNG

(11) **EP 0 911 386 A2**
(43) Veröffentlichungstag der Anmeldung: **28.04.1999**
(21) Anmeldenummer: 98250364.1
(22) Anmeldetag: 16.10.1998
(51) Int. Cl.: C12M 1/04, C12M 1/00

(54) **Bioreaktor mit U-förmigen Reaktorelementen**

(30) Priorität: 17.10.1997 DE 19747994
(71) Anmelder: Torsten Steinau Verfahrenstechnik Berlin, 13353 Berlin (DE)
(72) Erfinder: Buchholz, Rainer, Prof. Dr., 14641 Berlin (DE); Gerbsch, Norbert, Dr., 12309 Berlin (DE); Walter, Chistian, 12359 Berlin (DE); Steinau, Torsten, 13357 Berlin (DE)
(74) Vertreter: Jungblut, Bernhard Jakob

(57) **Zusammenfassung**

Die Erfindung betrifft einen Bioreaktor (1) zur Kultivierung von Mikroorganismen und/oder Pflanzenzellen mit einer Mehrzahl von zumindest für den sichtbaren Wellenlängenbereich des Lichts transparenten und im wesentlichen vertikal angeordneten Röhren (2), wobei die Röhren (2) die Mikroorganismen bzw. Pflanzenzellen in einer wäßriger Suspension enthalten und wobei die wäßrige Suspension in Richtung der Längserstreckung der Röhren (2) transportierbar ist. Die Erfindung ist dadurch gekennzeichnet, daß der Bioreaktor (1) zumindest ein Reaktormodul (3) aufweist, daß die Röhren (2) eines Reaktormoduls (3) als eine Mehrzahl von U-förmigen und an beiden Enden der Schenkel offenen Reaktorelementen (4) ausgebildet sind, und daß die beiden Enden der Schenkel der Reaktorelemente (4) an einen Reaktorkopf (5) angeschlossen sind, welcher einen Biomassesammelraum bildet, wobei im unteren Bereich eines Reaktorelements (4) Mittel (6) zum Transport der Suspension mit der Maßgabe eingerichtet sind, daß die Suspension in einem Schenkel des Reaktorelements (4) im wesentlichen nach unten transportierbar und in dem anderen Schenkel des Reaktorelements (4) im wesentlichen nach oben transportierbar ist.

## Beschreibung

Die Erfindung betrifft einen Bioreaktor zur Kultivierung von Biomasse mit einer Mehrzahl von im wesentlichen vertikal angeordneten Röhren, wobei die Röhren die Biomasse in einer wäßriger Suspension enthalten und wobei die wäßrige Suspension in Richtung der Längserstreckung der Röhren transportierbar ist. - In Bioreaktoren wird Biomasse aus Mikroorganismen bzw. Zellkulturen hergestellt bzw. vermehrt. Unter den Begriff der Mikroorganismen fallen z.B. Bakterien, Pilze Hefen und Algen. Dabei befindet sich die Biomasse in einer wäßrigen Suspension, welche neben Wasser auch gelöste Nährstoffe für die Biomasse enthalten kann. Weiterhin können gasförmige Nährstoffe, im Falle von zur Photosynthese befähigter Biomasse Kohlendioxid, in die Suspension eingeleitet werden. Aus Gründen der Sterilität empfiehlt es sich meist, einen Bioreaktor als geschlossenes System aufzubauen und die Bauelemente des Bioreaktors stoffschlüssig oder mit Dichtungselementen miteinander zu verbinden. Handelt es sich bei der Biomasse um Mikroorganismen bzw. Zellen, die zur Photosynthese befähigt sind, so sind die Röhren meist transparent, jedenfalls zumindest in Teilbereichen, ausgeführt. Durch die Wandungen der Röhren kann dann Licht die Suspension durchstrahlen oder in diese einstrahlen mit der Folge, daß die Biomasse im Wege der Photosynthese aus den Nährstoffen Energie gewinnt und sich vermehrt. In der Praxis wird hierbei oft das natürliche Tageslicht genutzt. Es können aber auch künstliche Lichtquellen eingesetzt werden, wobei Optimierungen hinsichtlich der Wellenlänge, der Intensität und der Verteilung des Lichts in Abstimmung auf den Bioreaktor und/oder die Biomasse getroffen werden können. Bei der Photosynthese entsteht Sauerstoff, welcher entfernt werden muß, bevor seine Konzentration für die Biomasse toxische Grenzwerte erreicht. Weiterhin empfiehlt es sich, zwecks hoher Vermehrungsrate die Temperatur der Suspension einzustellen bzw. zu regeln.

Bioreaktoren für zur Photosynthese befähigte Biomasse sind in den verschiedensten Ausführungen bekannt. Dabei sind die Röhren zur Erreichung einer hohen Produktionsrate im wesentlichen horizontal angeordnet und bis zu mehreren 100m lang. Meist bilden mehrere solcher Röhren den Bioreaktor. Bioreaktoren diesen Aufbaus nutzen in der Regel das Tageslicht zur Photosynthese. Als Beispiel für Bioreaktoren diesen Aufbaus wird auf die Literaturstelle WO 95/06111 verwiesen. Die insofern bekannten Bioreaktoren sind aufgrund der großen Längs- erstreckung der Röhren aufwendig im Platzbedarf und bei der Herstellung. Zudem sind sie anfällig gegen Beschädigungen, sei es durch höhere Gewalt oder durch mutwillige Beschädigungen. Wird eine Röhre undicht, so ist die gesamte darin enthaltene und wegen der Länge der Röhre eine sehr große Menge bildende Biomasse verloren.

Ein Bioreaktor des eingangs genannten Aufbaus ist aus der Literaturstelle RU 2019564 bekannt. Bei diesem Bioreaktor ist eine einzelne Röhre eingerichtet, welche nicht gerade, sondern schlangenlinienförmig in einer vertikalen Ebene angeordnet ist. Insofern wird bei gleicher effektiver Röhrenlänge ein reduzierter Platzbedarf erreicht. Ansonsten treffen jedoch auch die vorstehend bereits erläuterten Nachteile. Zudem sind relativ aufwendige Mittel zum Transport der Suspension erforderlich, nämlich geeignete Pumpenaggregate.

Demgegenüber liegt der Erfindung das technische Problem zugrunde, einen Bioreaktor anzugeben, der bei hoher Produktivität einen weiter verringerten baulichen Raumbedarf erfordert und von einfachem Aufbau ist. Der Erfindung liegt weiterhin das technische Problem zugrunde, einen Bioreaktor anzugeben, welcher aus einem Bauelementesatz mit wenigen, jeweils gleiche Elemente enthaltenden Bauelementegruppen auf einfache Weise aufgebaut und variabel dimensioniert werden kann.

Zur Lösung dieser technischen Probleme lehrt die Erfindung, daß der Bioreaktor zumindest ein Reaktormodul aufweist, daß die Röhren eines Reaktormoduls als eine Mehrzahl von U-förmigen und an beiden Enden der Schenkel offenen Reaktorelementen ausgebildet sind, und daß die beiden Enden der Schenkel der Reaktorelemente an einen Reaktorkopf angeschlossen sind, welcher einen Biomassesammelraum bildet, wobei im unteren Bereich eines Reaktorelements Mittel zum Transport der Suspension mit der Maßgabe eingerichtet sind, daß die Suspension in einem Schenkel des Reaktorelements im wesentlichen nach unten transportierbar und in dem anderen Schenkel des Reaktorelements im wesentlichen nach oben transportierbar ist. - Der Ausdruck Biomassesammelraum bezeichnet dabei eine Einrichtung, die auch als Kopfraum bezeichnet wird. In einem Biomassesammelraum kann Biomasse daher insbesondere auch durchmischt bzw. (teil-) homogenisiert werden. In einem Biomassesammelraum kann auch ein Totvolumen bzw. ein Gasphasenraum eingerichtet sein um beispielsweise Schaumbildung der Biomasse beherrschen zu können. Ein Reaktorelement kann einstückig oder mehrstückig ausgebildet sein.

Mit anderen Worten ausgedrückt ist erfindungsgemäß gleichsam durch Parallelschaltung mehrerer Abschnitte einer langen Röhre eine stark verkürzte Bauform erreicht worden bei hoher Produktivität eines Reaktormoduls. Der Stand der Technik arbeitet demgegenüber gleichsam mit einer Serienschaltung von Abschnitten zur Erhöhung der Produktivität. Weiterhin kann eine nochmals beträchtliche Verringerung des baulichen Raumbedarfs gegenüber einer in einer Ebene schlangenförmig verlaufenden Röhre dadurch erreicht werden, daß sich die Reaktorelemente bündeln lassen. Ein Reaktormodul kann also, in einer horizontalen Schnittebene betrachtet, mehrere Reaktorelemente in jeder horizontalen Raumrichtung verteilt aufweisen. Es lassen sich die Reaktorelemente, in einer horizontalen Schnittebene betrachtet, insbesondere in einem Raster anordnen. Aufgrund der Parallelschaltung führt eine Beschädigung eines Reaktorelements auch nicht zum Verlust der gesamten Biomasse eines Reaktormoduls, sondern allenfalls der in einem Reaktorelement befindlichen Biomasse. Ein wesentlicher weiterer Vorteil besteht darin, daß ein erfindungsmäßer Bioreaktor sich aus den Materialien Glas und/oder Glas fertigen läßt, was bei den Freilandreaktoren aus technischen und/oder wirtschaftlichen nicht zweckmäßig ist. Dies ermöglicht es einen erfindungsgemäßen Reaktor nicht nur mittels Druckbeaufschlagung, sondern zusätzlich oder stattdessen mittels thermischer Sterilisierung, beispielsweise Streichdampfbeaufschlagung. Eine solche thermische Sterilisierung ist bei Reaktoren aus Kunststoffmaterialien dagegen nicht ohne weiteres möglich.

Im Falle der Kultivierung zur Photosynthese befähigter Mikroorganismen bzw. Zellen sind zumindest Teilbereiche der Reaktorelemente transparent. Es können im Bedarfsfalle auch künstliche Lichtquellen außerhalb eines Reaktormoduls angeordnet sein; künstliche Lichtquellen können aber auch, ggf. zusätzlich, zwischen den Reaktorelementen eines Reaktormoduls eingerichtet sein, beispielsweise entsprechend einem Raster der Reaktorelemente. Dann sind, sofern vorgesehen, außerhalb des Reaktormoduls angeordnete Lichtquellen zweckmäßigerweise entsprechend dem Raster bzw. dessen translatorischer Fortsetzung angeordnet. Als Lichtquellen kommen beispielsweise Leuchtstoffröhren in Frage. Die Leuchtstärke der Lichtquellen kann steuer- und/oder regelbar sein. Es können nichttransparente Teilbereiche im Rahmen eines an sich transparenten Reaktorelements vorgesehen sein, beispielsweise wenn eine Temperierung mittels Doppelmantelwärmetauscher gewünscht ist.

Eine bevorzugte Weiterbildung der Erfindung ist dadurch gekennzeichnet, daß der Reaktorkopf strömungstechnisch mit der Maßgabe ausgebildet ist, daß eine gute Durchmischung der im Reaktorkopf befindlichen Suspension erfolgt. Solche Maßnahmen im Rahmen des Reaktorkopfes sind mittels einfacher Überlegungen und ggf. Experimente für den Durchschnittsfachmann einrichtbar. Wesentlich dabei beispielsweise, daß die Reaktorelemente hinsichtlich der Strömungsrichtung der Suspension darin so an den Reaktorkopf angeschlossen werden, daß in den Reaktorkopf einströmende Suspension nicht auf direktem Wege wieder in ein Reaktorelement ausströmt, sondern an einer Umwälzbewegung der Suspension im Reaktorkopf teilnimmt. Dies läßt sich beispielsweise dadurch realisieren, daß der Reaktorboden als Klöpperboden mit einer vertikalen Symmetrieachse ausgebildet ist, und daß die Anschlüsse zumindest einiger Reaktorelemente auf Radien zu der Symmetrieachse angeordnet sind. Dadurch wird ein Umwälzbewegung in dem Reaktorkopf von innen nach außen oder umgekehrt eingerichtet.

Eine besonders vorteilhafte und kompakte Ausführungsform der Erfindung ist dadurch gekennzeichnet, daß acht Reaktorelemente eingerichtet sind, daß der Reaktorkopf als Klöpperboden mit im wesentlichen rotationssymmetrisch zu einer senkrechten Symmetrieachse und konvex ausgeformten Reaktorkopfunterteil ausgebildet ist und daß Anschlüsse für die Schenkel der Reaktorelemente mittig zur Symmetrieachse in einem 4 x 4 Raster angeordnet sind, wobei vier Reaktorelemente in Projektion entlang der Symmetrieachse und mit Blick auf U-Bögen der Reaktorelemente radial zur Symmetrieachse und vier Reaktorelemente orthogonal zu Radien der Symmetrieachse angeordnet sind, wobei die Transportrichtung der Suspension in den radial angeordneten Reaktorelementen bei Blick auf die U-Bögen radial nach außen weist und wobei die Symmetrieachse hinsichtlich der Anordnung der Reaktorelemente sowie der Transportrichtungen in allen Reaktorelementen bei Blick auf die U-Bögen eine 4-zählige Symmetrieachse ist. An dieses spezielle Ausführungsbeispiel lassen sich selbstverständlich entsprechende Variationen anschließen. Beispielsweise kann die Symmetrieachse eine andere Wertigkeit, beispielsweise 3-zählig, 5-zählig, 6-zählig usw. sein. Entsprechend kann die Anzahl der Reaktorelemente kleiner oder größer als acht sein, jeweils angepaßt an die ausgewählte Symmetrie. Wichtig ist lediglich, daß zumindest ein Teil der Reaktorelemente radial oder zumindest mit einer radialen Orientierungskomponente zur Symmetrieachse angeordnet sind. Sofern Lichtquellen vorgesehen sind, können diese entsprechend dem Raster angeorndnete Leuchtröhren sein, deren Längserstreckung parallel zu den Schenkeln der Reaktorelemente verläuft. Bei einem 4 x 4 Raster der Anschlüsse wäre ein solches Lichtquellenraster zweckmäßigerweise ein 5 x 5 Raster, so daß jeder Schenkel eines Reaktorelements von 4 Lichtquellen umgeben ist.

Im einzelnen kann ein erfindungsgemäßer Bioreaktor dadurch weitergebildet sein, daß die Mittel zum Transport der Suspension Gaseinleitungsdüsen, vorzugsweise zur Einleitung von Trägergasen wie Luft oder Stickstoff und/oder von Nährstoffgasen wie Kohlendioxid sind und daß die Mittel zum Transport der Suspension im Bereich eines Schenkels eines U-Bogens oder im benachbarten Bereich eines Schenkels des Reaktorelements angeordnet sind. Mit dieser Anordnung wird die Suspension durch aufsteigendes Gas aufwärtsbewegt und entsprechend Suspension im anderen Schenkel des Reaktorelements abwärts bewegt. Vorzugsweise weisen alle Reaktorelemente eines Reaktormoduls jeweils eine Gaseinleitungsdüse auf. Die Gaseinleitungsdüsen können aber auch im Scheitelpunkt der U-Bögen oder sogar im strömungstechnisch absteigenden Schenkel eines Reaktorelements vorgesehen sein. Dann sind allerdings zusätzliche Maßnahmen zum "Anwerfen" der Suspension erforderlich. Bei entsprechender Einstellung der Gasmenge behält dann die Suspension trägheitsbedingt die anfangs hervorgerufene Bewegung bei, selbst bei im absteigenden Schenkel angeordneter Gaseinleitungsdüse. Im einzelnen weist der Reaktorkopf Mittel zur Ableitung und/oder Absorption bzw. Adsorption von Stoffwechselproduktgasen der Biomasse (Mikroorganismen bzw. Pflanzenzellen) auf. Hier kann auf übliche Einrichtungen zurückgegriffen werden, die dem Fachmann wohl vertraut sind und hier nicht näher erläutert zu werden brauchen. Ebenso empfiehlt es sich, daß der Reaktorkopf Mittel zur Temperierung der Suspension aufweist. Dies können beispielsweise Wärmetauscherschlangen sein, welche in einer horizontalen Ebene verlaufend im Inneren des Reaktorkopfs angeordnet sind.

Von selbstständiger erfinderischer Bedeutung sind Ausführungsformen der Erfindung, in welchen mehrere Reaktormodule miteinander verknüpft sind. Hiermit läßt sich die Produktivität entsprechend der Anzahl an Reaktormodulen vervielfachen. Im Kern entspricht dies der Anordnung mehrerer (langer) Röhren gemäß dem Stand der Technik. Allerdings ist es möglich, Reaktormodule zweidimensional rasterartig nebeneinander anzuordnen und so zu einer besonders hohen Packungsdichte der Reaktorelemente zu kommen. Ein allgemeines Beispiel für einen solchen Bioreaktor ist dadurch gekennzeichnet, daß der Bioreaktor zumindest zwei Reaktormodule aufweist, wobei die Reaktormodule dadurch miteinander verbunden sind, daß zumindest zwei Verbindungsreaktorelemente eingerichtet sind, welche zumindest je eines der Reaktorelemente eines Reaktormoduls ersetzen, wobei die Schenkel eines Verbindungsreaktorelements jeweils verschiedenen Reaktormodulen zugeordnet sind. Eine Optimierung hinsichtlich der Verbindungsreaktorelemente ist beispielsweise bei einem Bioreaktor aus zwei benachbarten Reaktorelementen erreichbar, wenn Reaktormodule nach Anspruch 3 verwendet und hinsichtlich der Rasteranordnung der Anschlüsse für Schenkel der Reaktorelemente parallel zueinander angeordnet sind, wobei in jedem Reaktormodul drei unmittelbar einander gegenüberliegende Reaktorelemente verschiedener Reaktormodule entfernt sind, wobei die Anschlüsse für Schenkel der Reaktorelemente, welche in den jeweils einander unmittelbar gegenüberliegenden Rasterreihen der Reaktormodule liegen, jeweils mit einem Verbindungsreaktorelement verbunden sind und wobei die verbleibenden zwei Anschlüsse jedes Reaktormoduls mit einem Reaktorelement verbunden sind.

Zur Lösung der genannten technischen Probleme lehrt die Erfindung auch einen Bauelementesatz zur Herstellung eines Bioreaktors nach einem der Ansprüche 1 bis 7, der dadurch gekennzeichnet ist, daß der Bauelementesatz folgende Bauelementegruppen umfaßt: A) zumindest zwei zylinderförmige Bioreaktorröhren gleicher Länge, B) zumindest ein an ein Ende verschiedener Bioreaktorröhren anschließbares U-Bogenelement, C) zumindest ein Reaktorkopfunterteil mit Anschlüssen für Bioreaktorröhren und einen Reaktorkopfdeckel. - Aus jeweils zwei Bioreaktorröhren der Gruppe A) und einem U-Bogenelement der Gruppe B) läßt sich ein Bioreaktorelement herstellen. Mehrere so hergestellte Bioreaktorelemente lassen sich dann an ein Reaktorkopfunterteil der Bauelementegruppe C) anschließen. Dabei versteht es sich, daß die Bauelemente der Gruppen A) bis C) jeweils miteinander kompatible Anschlüsse, d.h. z.B. baugleiche Flansche oder komplementäre Anschlüsse, aufweisen, welche ggf. auch mit Dichtungselementen verbunden werden können. Insofern umfaßt der Bauelementesatz ggf. auch Anschlußübergangsstücke, Dichtelemente, Verbindungs- und Befestigungsmittel und dergleichen. Grundsätzlich sind zwar alle Bauelemente der Gruppe A) gleich lang, wenn jedoch Reaktorelemente unterschiedlicher Bauhöhe gewünscht sind, umfaßt die Gruppe A) aber auch Untergruppen mit (gradzahligen Mengen an) Bioreaktorröhren verschiedener Länge. Insbesondere, wenn beispielsweise einer oder mehrere Bioreaktoren nach Anspruch 3 oder Anspruch 7 hergestellt werden sollen, besteht die Bauelementegruppe B) aus zumindest zwei, vorzugsweise drei Bauelementeuntergruppen B1), B2) und ggf. B3), wobei sich die Bauelementeuntergruppen B1) und B2) dahingehend unterscheiden, daß der Abstand der Schenkel der U-Bogenelemente 12 der jeweiligen Bauelementuntergruppen ein Verhältnis von 1 : 2^{0,5} bildet. Der Abstand der Schenkel der Bauelementeuntergruppe B3) richtet sich nach dem Abstand, den zwei Reaktormodule aufweisen sollen. Mit der Einrichtung eines Reaktorkopfunterteils und eines Reaktorkopfdeckels ist es natürlich gleichwertig einen einstückigen als Reaktorkopf ausgebildeten Behälter vorzusehen.

Im folgenden wird die Erfindung anhand von lediglich Ausführungsbeispielen darstellenden Figuren näher erläutert. Es zeigen in schematischer Darstellung:
- Fig. 1:: einen erfindungsgemäßen Bauelementesatz zur Herstellung eines Bioreaktors,
- Fig. 2:: ein erfindungsgemäßes Reaktormodul in Seitenansicht,
- Fig. 3:: den Gegenstand der Fig. 2 in Projektion auf die Ebene A-A,
- Fig. 4:: einen erfindungsgemäßen Bioreaktor aus zwei Reaktormodulen in einer Ansicht entsprechend jener der Fig. 3 und
- Fig. 5:: einen erfindungsgemäßen Bioreaktor aus vier Reaktormodulen in einer Ansicht entsprechend jener der Fig. 3.

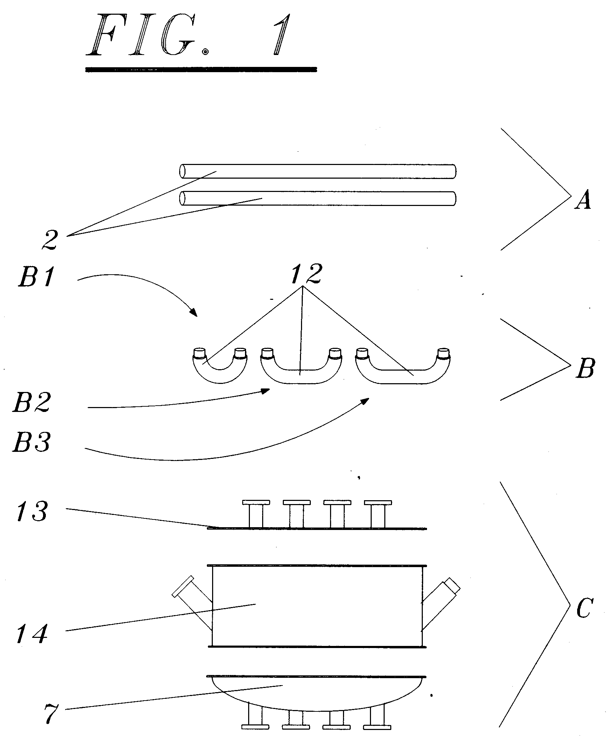

Der in der Fig. 1 dargestellte Bauelementesatz umfaßt folgende Bauelementegruppen: A) eine Mehrzahl zylinderförmiger Bioreaktorröhren gleicher Länge (11), und eine Bauelementegruppe B) aus jeweils mehreren, verschiedenen, an ein Ende verschiedener Bioreaktorröhren 11 anschließbaren U-Bogenelementen, wobei die Bauelementegruppe B) drei Bauelementeuntergruppen B1), B2) und B3) umfaßt, wobei sich die Bauelementeuntergruppen B1) und B2) dahingehend unterscheiden, daß der Abstand der Schenkel der U-Bogenelemente 12 der jeweiligen Bauelementuntergruppen ein Verhältnis von 1 : 2^{0,5} bildet. Der Abstand der Schenkel der U-Bogenelemente 12 der Bauelementeuntergruppe B3) ist im Ausführungsbeispiel größer als jener der Schenkel der U-Bogenelemente 12 der Bauelementeuntergruppen B1) und B2). Der Bauelementesatz umfaßt weiterhin eine Bauelementegruppe C) mit zumindest einem Reaktorkopfunterteil 7 mit Anschlüssen für Bioreaktorröhren 11. In der Darstellung der Fig. 1 weist die Bauelementgruppe C) neben dem Reaktorkopfunterteil 7 noch weitere Bauelemente auf. Dies sind ein im wesentlichen planer Reaktorkopfdeckel 13 und ein im wesentlichen zylinderförmiger Aufsatzbehälter 14. Bei den gezeigten Bauelementen der Bauelementegruppe C) lassen sich so die verschiedensten Reaktorkopfvolumina realisieren. Bei der Verwendung eines Reaktorkopfunterteils 7 mit einem Reaktorkopfdeckel 13 wird das kleinste Volumen erhalten. Ein verdoppeltes Volumen erhält man, wenn man ein Reaktorkopfunterteil 7 auf ein anderes Reaktorkopfunterteil 7 umgekehrt aufsetzt. Mittels eines oder mehrerer Aufsatzbehälter 14 kann das Volumen entsprechend weiter erhöht werden, wobei der obere Abschluß wiederum entweder durch ein umgekehrtes Reaktorkopfunterteil 7 oder durch einen Reaktorkopfdeckel 13 gebildet werden kann. Es versteht sich dabei, daß nicht benötigte, obenliegende Flansche mittels beispielsweise Blindstücken verschlossen werden sollten. Zumindest einige der obenliegenden Flansche lassen sich jedoch zur Einführung oder zur Entnahme von Biomasse, zur Entnahme von Gas oder zur Durchführung von Sensorenleitungen oder Wärmetauscherleitungen nutzen. Hinsichtlich der Sensorik können beispielsweise Temperatursensoren und/oder pH-Sensoren und/oder Drucksensoren vorgesehen sein.

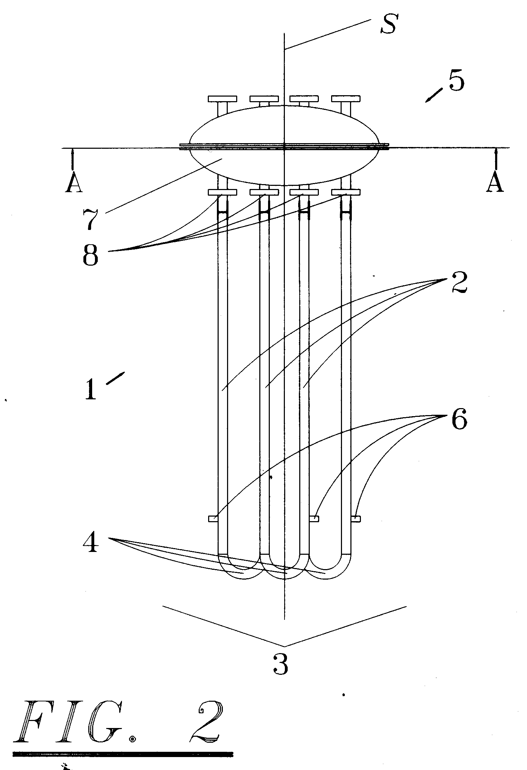

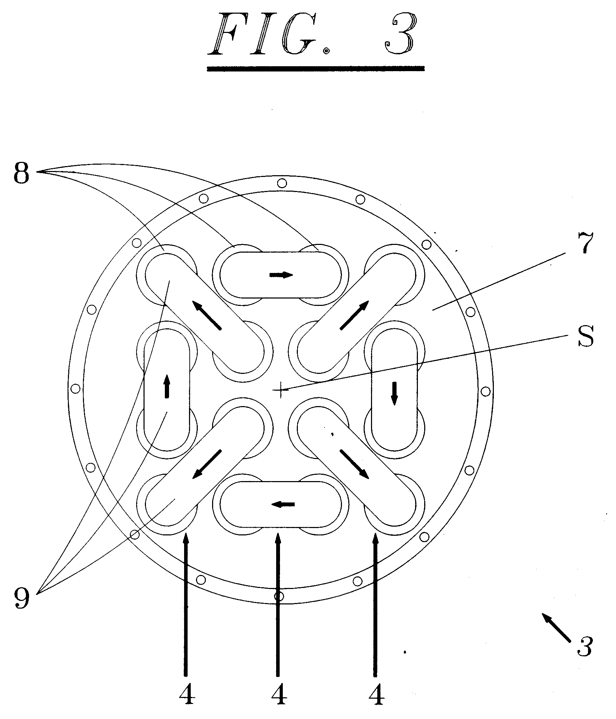

In den Figuren 2 und 3 erkennt man einen aus Bauelementen der Fig. 1 zusammengesetzten Bioreaktor 1 zur Kultivierung von Biomasse mit einer Mehrzahl von im wesentlichen vertikal angeordneten Röhren 2, wobei die Röhren 2 die Biomasse in einer wäßriger Suspension enthalten und wobei die wäßrige Suspension in Richtung der Längserstreckung der Röhren 2 transportierbar ist. In diesem Ausführungsbeispiel besteht der Bioreaktor 1 einem Reaktormodul 3. Die Röhren 2 eines Reaktormoduls 3 sind als eine Mehrzahl von U-förmigen und an beiden Enden der Schenkel offenen Reaktorelementen 4 ausgebildet. Die beiden Enden der Schenkel eines Reaktorelements 4 sind an einen Reaktorkopf 5 angeschlossen, welcher einen Biomassesammelraum bildet. Im unteren Bereich eines Reaktorelements 4 sind Mittel 6 zum Transport der Suspension mit der Maßgabe eingerichtet, daß die Suspension in einem Schenkel des Reaktorelements 4 im wesentlichen nach unten transportierbar und in dem anderen Schenkel des Reaktorelements 4 im wesentlichen nach oben transportierbar ist. Diese Mittel 6 zum Transport der Suspension sind Gaseinleitungsdüsen zur Einleitung von Nährstoffgasen und/oder Trägergasen wie Luft bzw. Stickstoff. Die Mittel 6 zum Transport der Suspension sind im Bereich eines Schenkels eines U-Bogens 9 oder im dazu benachbarten Bereich eines Schenkels des Reaktorelements 4 anbringbar, im Ausführungsbeispiel ist die letztgenannte Alternative gewählt. Es versteht sich dabei, daß die Bauelemente der Bauelementegruppe A) antsprechende Anschlüsse aufweisen, die in den Figuren 1 und 3 bis 5 der Übersichtlichkeit halber nicht gezeichnet sind. Der Reaktorkopf 5 ist strömungstechnisch mit der Maßgabe ausgebildet ist, daß eine gute Durchmischung der im Reaktorkopf 5 befindlichen Suspension erfolgt. Im einzelnen sind acht Reaktorelemente 4 eingerichtet. Der Reaktorkopf 5 ist als Klöpperboden mit im wesentlichen rotationssymmetrisch zu einer senkrechten Symmetrieachse S und konvex ausgeformten Reaktorkopfunterteil 7 ausgebildet. Anschlüsse 8 für die Schenkel der Reaktorelemente 4 sind mittig zur Symmetrieachse S in einem 4 x 4 Raster angeordnet (Fig. 3). Vier Reaktorelemente 4 sind in Projektion entlang der Symmetrieachse S und mit Blick auf U-Bögen 9 der Reaktorelemente 4 radial zur Symmetrieachse und vier Reaktorelemente 4 orthogonal zu Radien der Symmetrieachse S angeordnet. Die Transportrichtung der Suspension in den radial angeordneten Reaktorelementen 4 bei Blick auf die U-Bögen 9 weist radial nach außen. Die Symmetrieachse S ist hinsichtlich der Anordnung der Reaktorelemente 4 sowie der Transportrichtungen in allen Reaktorelementen 4 bei Blick auf die U-Bögen 9 eine 4-zählige Symmetrieachse. Der Übersichtlichkeit halber nicht gezeichnet ist, daß der Reaktorkopf 5 Mittel zur Ableitung und/oder Absorption bzw. Adsorption von Stoffwechselproduktgasen der Mikroorganismen bzw. Pflanzenzellen aufweist, daß der Reaktorkopf 5 Mittel zur Temperierung der Suspension aufweist, und daß im Rahmen des Reaktorkopfes 5 eine übliche Sensorik eingerichtet ist.

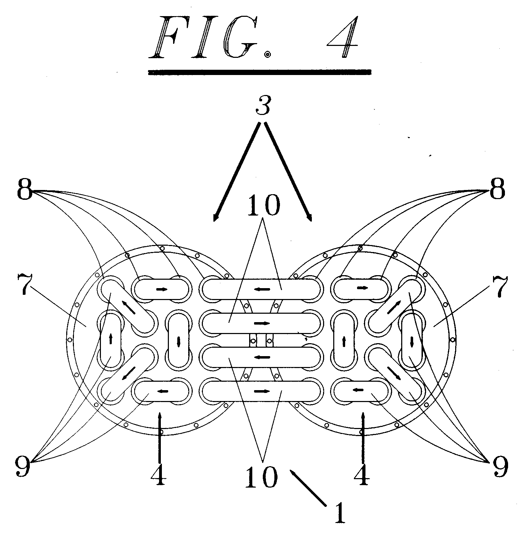

In der Fig. 4 ist dargestellt, daß in einem weiteren Ausführungsbeispiel der Erfindung der Bioreaktor 1 zumindest zwei Reaktormodule 3 aufweist, wobei die Reaktormodule 3 dadurch miteinander verbunden sind, daß zumindest zwei Verbindungsreaktorelemente 10 eingerichtet sind, welche zumindest je eines der Reaktorelemente 4 eines Reaktormoduls ersetzen, wobei die Schenkel eines Verbindungsreaktorelements 10 jeweils verschiedenen Reaktormodulen 3 zugeordnet sind. Im einzelnen sind zwei Reaktormodule 1 eingerichtet und benachbart angeordnet, wobei Reaktormodule 3 nach Anspruch 3 verwendet und hinsichtlich der Rasteranordnung der Anschlüsse 8 für Schenkel der Reaktorelemente 4 parallel zueinander angeordnet sind. In jedem Reaktormodul 3 sind drei unmittelbar einander gegenüberliegende Reaktorelemente 4 verschiedener Reaktormodule 3 entfernt, wie eine vergleichende Betrachtung der Figuren 3 und 4 ergibt. Die Anschlüsse 8 für Schenkel der Reaktorelemente 4, welche in den jeweils einander unmittelbar gegenüberliegenden Rasterreihen der Reaktormodule 3 liegen, sind jeweils mit einem Verbindungsreaktorelement 10 verbunden. Die verbleibenden zwei Anschlüsse 8 jedes Reaktormoduls 3 sind mit einem Reaktorelement 4 verbunden.

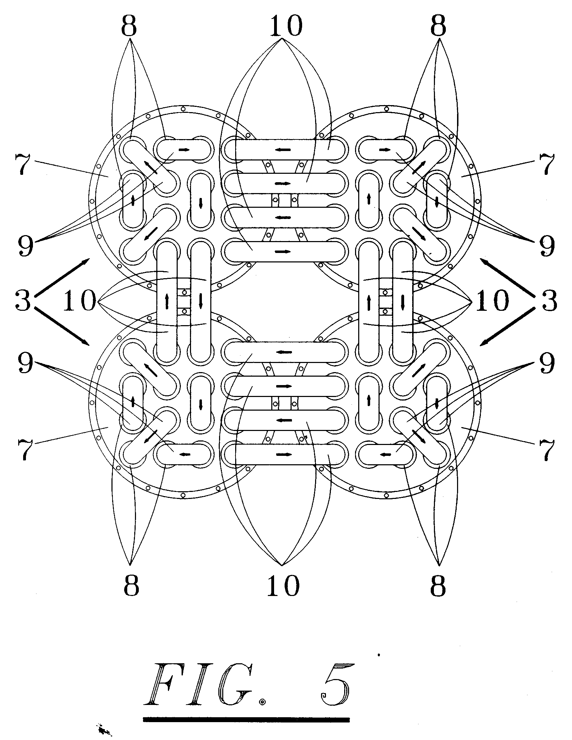

In der Fig. 5 ist schließlich eine Weiterbildung bzw. Erweiterung des Gegenstandes der Fig. 4 dargestellt. Durch vergleichende Betrachtung der Figuren 4 und 5 erkennt man, wie sich zwei Einheiten gemäß der Fig. 4 miteinander verbinden lassen. Aus einer vergleichender Betrachtung dieser beiden Figuren wird auch deutlich, daß sich zusätzliche Erweiterungen auf ganz entsprechende Weisen anschließen lassen. Im Ergebnis erhält man einen Bioreaktor 1 aus einer Mehrzahl von Reaktormodulen, welche in einem n x n oder in einem n x m Raster zueinander angeordnet sind. Dabei erfolgen die weiteren Verbindungen in einer Zeile bzw. in einer Spalte des Rasters in der in den Figuren 4 und 5 gezeigten Weise. Mit einem Bioreaktor gemäß der Figuren 4 oder 5 bzw. entsprechenden Erweiterungen wird aufgrund der besonderen gezeigten Verschaltung der Reaktormodule 3 untereinander auch eine besonders gute Homogenität der Biomasse in dem Bioreaktor 1 insgesamt erreicht. Hierbei ist auch - ebenso wie bei einem einzelnen Reaktormodul 3 - bedeutsam, daß in jedes der vergleichsweise kurzen Reaktorelemente 4 Gas eingeleitet wird und folglich Konzentrationsgradienten an Nährstoffgas und/oder Sauerstoff sich nicht so stark ausbilden können, wie bei Bioreaktoren aus dem Stand der Technik. Dies ist dabei völlig unabhängig von der effektiven Gesamtlänge an Röhren, die im Rahmen eines erfindungsgemäßen Bioreaktors eingerichtet ist.

## Patentansprüche

1. Bioreaktor (1) zur Kultivierung von Biomasse mit einer Mehrzahl von im wesentlichen vertikal angeordneten Röhren (2), wobei die Röhren (2) die Biomasse in einer wäßriger Suspension enthalten und wobei die wäßrige Suspension in Richtung der Längserstreckung der Röhren (2) transportierbar ist,
dadurch gekennzeichnet,
daß der Bioreaktor (1) zumindest ein Reaktormodul (3) aufweist,
daß die Röhren (2) eines Reaktormoduls (3) als eine Mehrzahl von U-förmigen und an beiden Enden der Schenkel offenen Reaktorelementen (4) ausgebildet sind, und
daß die beiden Enden der Schenkel der Reaktorelement (4) an einen Reaktorkopf (5) angeschlossen sind, welcher einen Biomassesammelraum bildet,
wobei im unteren Bereich eines Reaktorelements (4) Mittel (6) zum Transport der Suspension mit der Maßgabe eingerichtet sind, daß die Suspension in einem Schenkel des Reaktorelements (4) im wesentlichen nach unten transportierbar und in dem anderen Schenkel des Reaktorelements (4) im wesentlichen nach oben transportierbar ist.

2. Bioreaktor (1) nach Anspruch 1, dadurch gekennzeichnet, daß der Reaktorkopf (5) strömungstechnisch mit der Maßgabe ausgebildet ist, daß eine gute Durchmischung der im Reaktorkopf (5) befindlichen Suspension erfolgt.

3. Bioreaktor (1) nach Anspruch 2, dadurch gekennzeichnet, daß acht Reaktorelemente (4) eingerichtet sind, daß der Reaktorkopf (5) als Klöpperboden mit im wesentlichen rotationssymmetrisch zu einer senkrechten Symmetrieachse (S) und konvex ausgeformten Reaktorkopfunterteil (7) ausgebildet ist und daß Anschlüsse (8) für die Schenkel der Reaktorelemente (4) mittig zur Symmetrieachse (S) in einem 4 x 4 Raster angeordnet sind, wobei vier Reaktorelemente (4) in Projektion entlang der Symmetrieachse (S) und mit Blick auf U-Bögen (9) der Reaktorelemente (4) radial zur Symmetrieachse und vier Reaktorelemente (4) orthogonal zu Radien der Symmetrieachse (S) angeordnet sind, wobei die Transportrichtung der Suspension in den radial angeordneten Reaktorelementen (4) bei Blick auf die U-Bögen (9) radial nach außen weist und wobei die Symmetrieachse (S) hinsichtlich der Anordnung der Reaktorelemente (4) sowie der Transportrichtungen in allen Reaktorelementen (4) bei Blick auf die U-Bögen (9) eine 4-zählige Symmetrieachse ist.

4. Bioreaktor (1) nach einem der Ansprüche 1 bis 3, dadurch gekennzeichnet, daß die Mittel (6) zum Transport der Suspension Gaseinleitungsdüsen, vorzugsweise zur Einleitung von Trägergasen wie Luft oder Stickstoff und/oder von Nährstoffgasen wie Kohlendioxid sind und daß die Mittel (6) zum Transport der Suspension im Bereich eines Schenkels eines U-Bogens (9) oder im benachbarten Bereich eines Schenkels des Reaktorelements (4) angeordnet sind.

5. Bioreaktor (1) nach einem der Ansprüche 1 bis 4, dadurch gekennzeichnet, daß der Reaktorkopf (5) Mittel zur Ableitung und/oder Absorption bzw. Adsorption von Stoffwechselproduktgasen der Mikroorganismen bzw. Pflanzenzellen aufweist und daß der Reaktorkopf (5) Mittel zur Temperierung der Suspension aufweist.

6. Bioreaktor (1) nach einem der Ansprüche 1 bis 5, dadurch gekennzeichnet, daß der Bioreaktor (1) zumindest zwei Reaktormodule (3) aufweist, wobei die Reaktormodule (3) dadurch miteinander verbunden sind, daß zumindest zwei Verbindungsreaktorelemente (10) eingerichtet sind, welche zumindest je eines der Reaktorelemente (4) eines Reaktormoduls ersetzen, wobei die Schenkel eines Verbindungsreaktorelements (10) jeweils verschiedenen Reaktormodulen (3) zugeordnet sind.

7. Bioreaktor (1) nach einem der Ansprüche 3 bis 6, dadurch gekennzeichnet, daß zwei Reaktormodule (1) eingerichtet und benachbart angeordnet sind, wobei Reaktormodule (3) nach Anspruch 3 verwendet und hinsichtlich der Rasteranordnung der Anschlüsse (8) für Schenkel der Reaktorelemente (4) parallel zueinander angeordnet sind, wobei in jedem Reaktormodul (3) drei unmittelbar einander gegenüberliegende Reaktorelemente (4) verschiedener Reaktormodule (3) entfernt sind, wobei die Anschlüsse (8) für Schenkel der Reaktorelemente (4), welche in den jeweils einander unmittelbar gegenüberliegenden Rasterreihen der Reaktormodule (3) liegen, jeweils mit einem Verbindungsreaktorelement (10) verbunden sind und wobei die verbleibenden zwei Anschlüsse (8) jedes Reaktormoduls (3) mit einem Reaktorelement (4) verbunden sind.

8. Bauelementesatz zur Herstellung eines Bioreaktors (1) nach einem der Ansprüche 1 bis 7, dadurch gekennzeichnet, daß der Bauelementesatz folgende Bauelementegruppen umfaßt:
A) zumindest zwei zylinderförmige Bioreaktorröhren gleicher Länge (11),
B) zumindest ein an ein Ende verschiedener Bioreaktorröhren (11) anschließbares U-Bogenelement 12),
C) zumindest einem Reaktorkopfunterteil (7) mit Anschlüssen für Bioreaktorröhren (11) und einem Reaktorkopfdeckel (13).

9. Bauelementesatz nach Anspruch 8, dadurch gekennzeichnet, daß die Bauelementegruppe A) aus Bioreaktorröhren (11) gleicher Länge besteht, daß die Bauelementegruppe B) zumindest zwei, vorzugsweise drei Bauelementeuntergruppen B1), B2) und ggf. B3) umfaßt,
wobei sich die Bauelementeuntergruppen B1) und B2) dahingehend unterscheiden, daß der Abstand der Schenkel der U-Bogenelemente (12) der jeweiligen Bauelementuntergruppen ein Verhältnis von 1 : 2^{0,5} bildet.
